# EUROPEAN PATENT APPLICATION

(11) **EP 3 553 517 A1**
(43) Date of publication of application: **16.10.2019**
(21) Application number: 19160162.4
(22) Date of filing: 28.02.2019
(51) Int. Cl.: G01N 33/50

(54) **METHOD OF IDENTIFYING BIOACTIVE COMPOUNDS IN HERBALS**

(30) Priority: 14.04.2018 US 201862657722 P
(71) Applicant: Zhang, Jianyi, Front Royal, VA 22630 (US)
(72) Inventor: Zhang, Jianyi, Front Royal, VA 22630 (US)
(74) Representative: McQueen, Andrew Peter

(57) **Abstract**

The invention discloses a method by which potential bioactive compounds from herbals can be identified by regrouping after chromatography. The method comprises the steps of: selecting herbals with reports of therapeutic effects according to previous experience, extracting compounds by boiling or chemical methods, and separating compounds with the chromatography. The separated compounds are regrouped with different portions, which are further tested in vivo and/or in vitro to remove the inactive portions and repeating the same process until no more compounds can be removed, and the remaining compounds are synthesized or purified, which are made into various forms and utilized to treat diseases.

## Description

### CROSS-REFERENCE TO RELATED APPLICATIONS

The present application relates and claims priority to U.S. provisional patent application No. 62657722, filed on April 14, 2018.

### FIELD OF THE INVENTION

This invention relates to methods to isolate potential bioactive compounds from a mixture of herbals.

### ABBREVIATIONS

The terms "FDA" or "US FDA" refer to the Food and Drug Administration, a USA governmental agency which regulates food and drug in USA.

The term "HPLC" refers to high pressure liquid chromatography.

The term "LC-MS-MS" refers to liquid chromatography-mass-mass spectrometry.

The term "PBCs" refers to potential bioactive compounds.

The term "TCM" refers to Traditional Chinese Medicine.

### INTRODUCTION

Herbal medicine has a long history to be applied for treatment of various medical conditions. When ones talk about drug treatment, the first choice is Western medicine, most of them were developed in accordance with the FDA regulations in America. The advantage of this development is that his efficacy and safety can be fully proved, and its quality and quantity care well controlled.

Two scholars from Tufts University in Boston, USA, published their findings in 2014. They collected data of drug development ranged last few decades, time from basic science to clinical trials is around 11 years with $ 1.4 billion expenditure; time from the clinical trials to the FDA approval is about 7-8 years, which costs around $ 2.4 billion. So, it takes 18 years and $ 3.8 billion from basic science to FDA approval of a drug on average. For many Westerners, this is the only treatment of choice.

In the Asia and many third world countries, herbal medicine is the second choice. Traditional Chinese medicine (TCM) is the ultimate development of herbal medicine. Except for China, herbal medicine in other places is basically single herbal, TCM has its own theory, many times practitioners treat patients with mixture of 10-20 herbals.

There are numerous compounds even in one herbal, and many more in mixed herbals.

Nature of many these compounds are still unknown. Scientists have spent a lot of money and time to study bioactive compounds in herbals, only with little a success. Artemisinin is one for treatment of malaria.

There are many methods for studying medicinal medicine, such as high-pressure liquid chromatography (HPLC), gas-mass spectrometry (GC-MS), liquid - mass - mass spectrometry (LC-MS-MS), etc. However, these methods can only determine the structures of the chemical components, but they cannot clearly identify the efficacy and safety of the compounds. Therefore, how to identify safety and efficacy of compounds in herbals has always been a bottleneck in study of medicinal medicine.

There are many publications and patents in which chromatography were applied to separate compounds in herbals, but none of papers in which PBCs are selected and non-functional compounds removed.

### SUMMARY OF THE INVENTION

This invention provides a method for rapidly identifying PBCs by using regrouping after chromatography.

Chromatography is a laboratory technique for the separation of mixed compounds. The mixture is dissolved in a mobile phase, which carries it through another material called the stationary phase. The various constituents of the mixture travel at different speeds, leading them to separate. The separation is based on differential partitioning between the mobile and stationary phases.

Mixed herbals might have dozens of compounds, most of which are not related to the therapeutic effect. If one can remove most of the invalid ingredients, leaving only effective or possibly effective ones, it is possible to control quality and quantity of compounds like the Western medicine.

To achieve the above goal, following steps are taken:
(1): Based previous experience, select the combination of herbals for certain diseases to extract the compounds they contain. Extraction may be performed by boiling or chemical methods;
(2): Separate them by chromatography, group isolated compounds;
(3): Select proper vitro or vivo models and test grouped compounds to see these compounds effective.
(4): Re-group and repeat the process until no more non-PBCs removed;
(5): The bioactive compounds found are made into various forms of medicines for treating patients;
(6): Mix other compounds into various forms of medicines for treating patients;
(7): Mix PBCs with other compounds in herbal or Western medicine to treat patients based on their illnesses.

The invention provides a method of identifying potential bioactive compounds of herbals, including the steps of:
selecting a herbal or mixed herbals based on previous experience; preparing selected herbals to extract all chemical compounds, concentrating compounds, applying concentrated solutions on chromatography and collecting output in different tubes; combining solutions in several portions; testing potential bioactivity with proper animal or other biological models, only keeping tubes that contains potential bioactive compounds (PBCs); repeating the above steps until no more removal possible; chemically analyzing remaining PBCs; preparing treatment regimens based on the ratio with PBCs; treating patients with combination of PBCs as food supplements or medications; adjusting amount of PBCs according patient's responses, adding or deleting other compounds for improvement of treatments.

The bioactive property for which the PBCs are tested may be an activity against a pathogenic organism such as a bacterium, a virus, a yeast or fungus, or a parasite.

Alternatively, the PBCs may be tested for a bioactive property that is effective for the treatment of a medical condition which is not due to foreign biological organism, such as hypertension, diabetics, cancers, etc.

In further aspects, the invention provides bioactive compounds identified by the disclosed method; bioactive compounds identified by the disclosed method for use in the treatment of a disease; and pharmaceutical formulations comprising said bioactive compounds and one or more pharmaceutically acceptable carriers, binders or excipients.

### DETAILED DESCRIPTION

Embodiments of the invention will now be described by way of example, with reference to the enclosed drawings, of which:

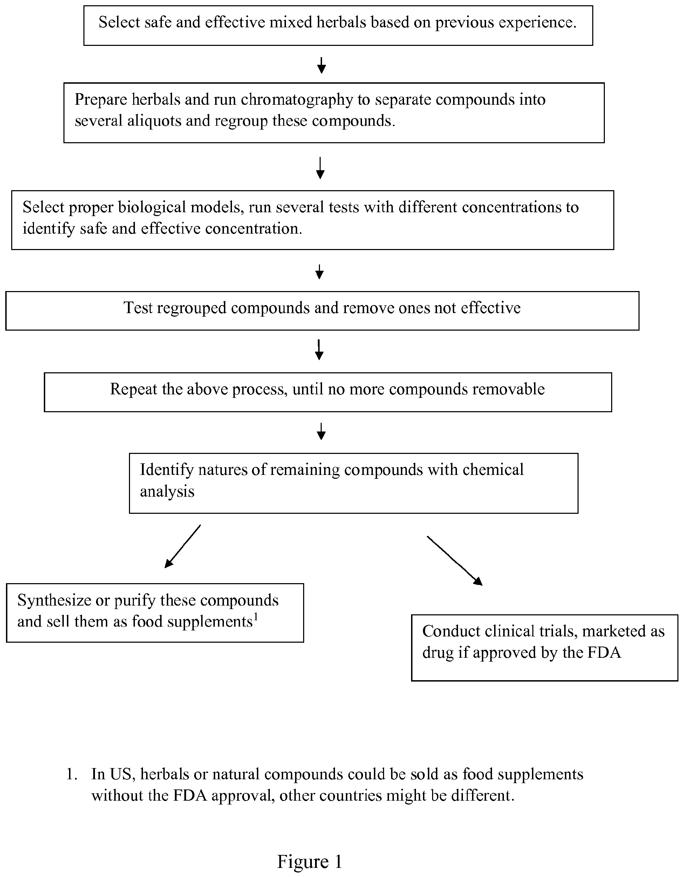

Figure 1 is a schematic showing the process of detection of potential bioactive compounds according to an embodiment of the invention.

### Embodiment 1. Discovery of PBCs with anti-opioid addiction activity in herbals with the invention.

It is reported that mixture of six herbals (Salvia miltiorrhiza 140-gram, Anemone altaicae 90 gram, Drynaria fortunei 30-gram, Cortex albizzia 30 gram, Polygonum Multiflorum 20 gram, Borneol 3.5 gram).

Mix the six herbs with 1,500 cc water, infuse for 30 minutes, boil them for 60 minutes at 100°, and pour the water into ajar, and repeat the above process one more time. Heat the collected water containing all herbal compounds in an oven at 100° until only 5-10 cc.

Analysis of the above solution by HPLC shows 61 compounds, the liquid obtained by column chromatography is divided into ten parts, and each group (compounds) are numbered as follows:

**Table 1**

| | | | |
|---|---|---|---|
| Group 1: 1-6 | Group 2: 7-12 | Group 3: 13-18 | Group 4: 19-24 |
| Group 5: 25-30 | Group 6: 31-36 | Group 7: 37-42 | Group 8: 43-48 |
| Group 9: 49-54 | Group 10: 55-61 | | |

The first test: determine safe amount of herbal compounds

Analysis of the collected solution by HPLC shows the 10^{th} peak prominent, which will be used as the reference one, diluted as 10 mg/ml. Mice will be initiated with morphine at 1mg/ml for 21 days.

The second test: use separate groups of compounds to test effectiveness in mice. The definition of effective.

**Table 2**

| | Compounds | Effective¹ | Conclusion |
|---|---|---|---|
| All groups | All 61 | Yes | Complete PBCs present after chromatography |
| Middle 2-9 | 7-54 | Yes | Complete PBCs present, the 1st and 10^{th} without PBCs |
| Middle 3-8 | 13-48 | Not | No complete PBCs, complete PBCs present in groups 2 & 9 |
| Middle 4-7 | 19-42 | Not | No complete PBCs |
| Morphine | 0 | Yes | Positive control |
| Saline | 0 | Not | Negative control |

| | | | |
|---|---|---|---|
| 1. Ten mice in each group. | | | |

From the above test, it is shown that group 2 and 9 contains complete PBCs, which have compounds 7-12 and 49-54. Regroup these compounds and perform the 3^{rd} test by using portions of compounds in the test. The result is as follows:

**Table 3**

| | compounds | Effective | Conclusion |
|---|---|---|---|
| Groups 2 & 9 | 7-12, 49-54 | Yes | Repeat the first test |
| Regroup-1 | 7, 8, 49, 50 | No | No complete PBCs |
| Regroup-2 | 9, 10, 51, 52 | Effective | With complete PBCs |
| Regroup-3 | 11, 12, 53, 54 | Not | No complete PBCs |
| Morphine | 0 | Yes | Positive control |
| Saline | 0 | Not | Negative control |

From the test 3, it is shown that compounds 9, 10, 51, 52 might be PBCs. Ones can continue to test these four compounds to see any further elimination possible. Analysis of these four compounds by LC-MS-MS shows commercial availability of the compounds 9 and 51, these compounds 10, 52 are obtained by purification or synthesis.

Patients with opioid addiction will be treated with mixture of compounds 9, 10, 51, 52 by ratio with stabilizers or vitamins.

### Embodiment 2. Discovery of PBCs with anti-E. Coli activity in herbals by chromatography

Complicated urinary tract infections (UTIs) may occur in all age groups and are frequently associated with either structural or functional urinary tract abnormalities. Most frequent microorganism is E. Coli.

Herbal remedies contain as active ingredients parts of plants, or plant materials, or combinations thereof used to treat a multitude of ailments throughout the world (WHO, 2002).

The prescription was as follow: Tong Cao (Stachyurus himalaicus Hook.f.et Thoms) 20g, Hua Shi (Talcum) 15g, Chi Shao (Paeonia lactiflora Pall.) 15g, Hui Xiang (Foeniculum vulgare Mill.) 15g, Guan Gui (Cinnamomum cassia Presl) 15g, Li Zhihe (Litchi chinensis Sonn) 15g, Tian Kuizi (Semiaquilegia adoxoides (DC.) makino) 15g, Zihua Diding (Viola ycdoensis Mak.) 20g, Ju Mai (Dianthus superbus L.) 15g, Ma Chixian (Portulaca oleracea L) 50g, Pu Gongying (Herba taraxaci) 30g. The concoction was prepared by mixing the crude drugs in 800ml water, getting 200ml liquor after the drugs were decocted in 800ml water (100° for 30 mins twice).

There are 88 compounds with HPLC analysis, these compounds will be divided into 11 groups as the below:

**Table 4**

| | | | |
|---|---|---|---|
| Group 1: 1-8 | Group 2: 9-16 | Group 3: 17-24 | Group 4: 25-32 |
| Group 5: 33-40 | Group 6: 41-48 | Group 7: 49-56 | Group 8: 57-64 |
| Group 9: 65-72 | Group 10: 73-80 | Group 11: 81-88 | |

The 1^{st} test is to determine effective concentration

By HPLC analysis, the 20^{th} compound is stable, by which reference compound is selected. The first step is to lyophilize, and cold freeze all liquid aliquots together collected by chromatography into powder, then dilute the powder to make the compound 20 at 50 mg/ml. Mix agar with 1, 2, 4 ml of the solution, with final total volume 50 ml. Apply E. coli on the top of agar, put them into incubator at 37 C for 24 hours. It is found that anti-bacterial activity is shown when 2 and 4 ml of the solution are mixed with the agar.

The criteria of anti-bacterial activity are set with following standards: yes, if the colonies of E. *Coli* per ml with 90% close to one treated with Fluoroquinolone 1mg/kg, which is common antibiotics for treatment of E. coli infection.

The 2^{nd} test: Perform tests by grouping compounds in the following ways, the result is as below:

**Table 5**

| | Compounds | anti- bacterial activity | Conclusion |
|---|---|---|---|
| Whole 11 groups | 88 | Yes | Complete PBCs present with chromatography |
| groups 1-4 | 32 | Yes | Complete PBCs present |
| groups 5-8 | 32 | No | No complete PBCs |
| groups 9-11 | 24 | No | No complete PBCs |
| Fluoroquinolone | 0 | Yes | Positive control |
| Saline | 0 | No | Negative control |

From the above test, it is shown that group 1-4 contains complete PBCs, which has 32 compounds (1-32). Regroup these compounds and perform the 3^{rd} test by using portions of compounds in the test. The result is as follows:

**Table 6**

| | Compounds | anti-bacterial activity | Conclusion |
|---|---|---|---|
| Re-group-1 | 1-8 | No | No complete PBCs |
| Re-group-2 | 9-16 | No | No complete PBCs |
| Re-group-3 | 17-24 | No | No complete PBCs |
| Re-group-4 | 25-32 | No | No complete PBCs |
| Fluoroquinolone | 0 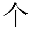 | Yes | Positive control |
| Saline | 0 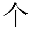 | No | Negative control |

From the above test, it is shown that regrouping compounds lead to loss anti-bacterial activity. Regrouping these compounds again and perform the 4^{th} test. The result is as follows:

**Table 7**

| | Compounds | anti- bacterial activity | Conclusion |
|---|---|---|---|
| Re-group-1 | 1-4, 9-12 | No | Without complete PBCs |
| Re-group-2 | 5-8, 13-16 | No | Without complete PBCs |
| Re-group-3 | 17-20, 25-28 | Yes | With complete PBCs |
| Re-group-4 | 21-24, 29-32 | No | Without complete PBCs |
| Fluoroquinolone | 0 | Yes | Positive control |
| Saline | 0 | No | Negative control |

From the above test, it is shown that compounds in re-grouping 3 contains anti-bacterial activity, which has 8 compounds (17-20, 25-28), regroup these compounds and perform the 5th test. The result is as follows:

**Table 8**

| | Compounds | Anti-bacterial activity | Conclusion |
|---|---|---|---|
| Re-group-1 | 17, 18, 25, 26 | No | Without complete PBCs |
| Re-group-2 | 19, 20, 25, 26 | No | Without complete PBCs |
| Re-group-3 | 17, 18, 27, 28 | Yes | With complete PBCs |
| Re-group-4 | 19, 20, 27, 28 | No | Without complete compounds |
| Fluoroquinolone | 0 | Yes | Positive control |
| Saline | 0 | No | Negative control |

Analysis of these four compounds by LC-MS-MS shows commercial availability of the compounds 18 and 27, these compounds 17, 28 are obtained by purification or synthesis.

Patients with the E. Coli infection may be treated with mixture of compounds 17, 18, 27, 28 by ratio with stabilizers.

Identified PBCs have not undergone these clinical trials required by the FDA, but they are shown to be effective in applications in animal models and/or microorganism studies. In the United States, they are dietary supplements that can be applied in human without FDA approval, as long as they are not marketed with medical applications.

Identified PBCs by this invention have all characteristics of TCM (low cost, multiple synergic compounds, short development time), which overcomes part of their shortcomings (lack of quality and quantity control). PBCs discovered by the invention could be "the third options". Table 9 below shows a comparison of these Western medicine, TCM and "the third options".

**Table 9. Comparison of the Three Ways of the Treatment**

| | TCM | Western Medicine | The third options |
|---|---|---|---|
| Safety | Gen-Speaking: good, not-well-documented | Gen-Speaking: good, well-documented | Gen-Speaking: good, well-documented in animal models |
| Efficacy | Some effective, but no clinical trials | Good | Effective in animal models², but no clinical trials |
| Qual. Control | Not good | Very good | Very good |
| No. of compounds | Many synergic | Only one unknown tested | A few synergic |
| Cost | Very low | Very high ($38 billions) | $100,000 to a few millions |
| Time | Very short | Very long (18 years) | A few weeks to a few months |
| FDA approved | No | Yes | Probably no |

The cost of research and development of the Western medicine is so high which makes patients pay very high to consume drugs. One-month cost of new cancer drug could be a few thousand US dollars. Even in rich country as the United States, many people can't afford it, not to mention others not so rich. Cost of research and development of PBCs by the invention will be greatly reduced, so are expense for patients who takes them. For patients who cannot afford to take medication due to prohibitive cost, "the third options" will be the "the first choice", or "the only options".

The above embodiments are a few examples of the invention, they are not intended to limit application of the invention. Any modifications, substitutions and improvements made consistent with the ideas and principles of the invention would be covered in the invention.

## Claims

1. A method of identifying potential bioactive compounds of herbals, including the steps of:
selecting an herbal or mixed herbal based on previous experience;
extracting compounds from the herbals, collecting the solution produced and concentrating it;
performing chromatography on the concentrated solution to separate the extracted compounds;
collecting output containing separated compounds in a series of sample tubes and combining selected samples to produce a plurality of portions, each portion containing a plurality (group) of compounds;
testing the portions for a desired bioactivity in an animal or other biological model and selecting only those portions that contain potential bioactive compounds (PBCs) for further testing;
re-grouping the compounds represented in the portions which tested positive for bioactivity and repeating the test and selection on the new groups;
repeating the re-grouping, test and selection steps as necessary to identify a group of compounds that contains the PBC or PBCs;
chemically analyzing the identified PBC or PBCs.

2. The method of claim 1, wherein the bioactivity is deleterious to a pathogen.

3. The method of claim 2, wherein the pathogen is a bacterium.

4. The method of claim 2, wherein the pathogen is a virus.

5. The method of claim 2, wherein the pathogen is a fungus or yeast.

6. The method of claim 2, wherein the pathogen is a parasite.

7. The method of claim 1 wherein the bioactivity is useful for the treatment of a medical condition which is not caused by a foreign biological organism.
